# EUROPEAN PATENT APPLICATION

(11) **EP 1 184 461 A2**
(43) Date of publication of application: **06.03.2002**
(21) Application number: 01307021.4
(22) Date of filing: 17.08.2001
(51) Int. Cl.: C12N 15/64, C12N 5/18, A01K 67/027, C12N 15/85

(54) **Creation of gene targeting vectors using homologous recombination in yeast**

(30) Priority: 25.08.2000 US 228467 P
(71) Applicant: Pfizer Products Inc., Groton, Connecticut 06340 (US)
(72) Inventor: Fisher, Katherine Elizabeth, Pfizer Global Reseach, Groton, Connecticut 06340 (US); Reaume, Andrew Gerard, Pfizer Global Reseach, Road Groton Connecticut 06340 (US)
(74) Representative: Hayles, James Richard

(57) **Abstract**

The present invention provides methods of preparing gene targeted mammalian cells having a targeted gene mutation, methods of making gene targeted mice, and gene targeting vectors that are useful in these methods.

SEQUENCE LISTING

<110> Pfizer Products Inc.

<120> Rapid Creation of Gene Targeting Vectors Using Homologous
Recombination in Yeast

<130> PC10667AGPR

<160> 11

<170> PatentIn version 3.0

<210> 1
<211> 30
<212> DNA
<213> Primer

<400> 1
cgcttcgaaa tcgctgggcc attctcatga 30

<210> 2
<211> 32
<212> DNA
<213> Primer

<400> 2
cgcatttaaa ttcttccgct tcctcgctca ct 32

<210> 3
<211> 21
<212> DNA
<213> Primer

<400> 3
gaccgcttat catcgataag c 21

<210> 4
<211> 32
<212> DNA
<213> Primer

<400> 4
cgcatttaaa tgaaggctct caagggcatc gg 32

<210> 5
<211> 49
<212> DNA
<213> Primer

<400> 5
cgcggatcct agcctggcca tccgggccat cgctgggcca ttctcatga 49

<210> 6
<211> 31
<212> DNA
<213> Primer

<400>. 6
cgcatcgatt tcgataagct ctgctttttg t 31


<210> 7
<211> 30
<212> DNA
<213> Primer

<400> 7
cgcttcgaac taccgggtag gggaggcgct 30


<210> 8
<211> 51
<212> DNA
<213> Primer

<400> 8
cgcagatctt ccgatggccg cagcggcctc tgatggaatt agaacttggc a 51


<210> 9
<211> 428
<212> DNA
<213> Multiple Cloning Site

<400> 9
ggccgctgct tccaggctaa cacttatata ggcattggta ctgagataca gcgagatacc 60

gtgtaactat aacggtccta aggtagcgaa ggctaacact tatataggca ttggtactga 120

gatacagcga gataccgtgc ggccgctgcg gccaattaac cctcactaaa gggaggccta 180

tgcatactag tccatgggat atccccgggg ttaacccgcg gctcgagatc gatgaattcg 240

gccggccgtt taaacggatc cggcgcgcct taattaaaag cttcatatgt ctagagtcga 300

cgagctcggt accgggcccg catgctggcc aaggcctaga tctccctata gtgagtcgta 360

ttatggcaaa cagctattat gggtattatg ggtaggccat ccgggccgcg gccgcttcca 420

tccgggcc 428

<210> 10
<211> 127
<212> DNA
<213> Primer

<400> 10
ccatcaacac gcgtctgcgt tcgaccaggc tgcgcgttct cgcggccata gcaaccgacg 60

tacggcgttg cgccctcgcc ggcagcaaga agccacggaa gtcatgtgcg cggaacccct 120

atttgtt 127


<210> 11
<211> 126
<212> DNA
<213> Primer

<400> 11
gagccagaac ggcgtcggtc acggcataag gcatgcccat tgttatctgg gcgcttgtca 60

ttaccaccgc cgcgtccccg gccgatatct caccctggtc gagatagcgc gggttccttc 120

cggtat 126

## Description

### FIELD OF THE INVENTION

The present invention relates to the development of gene targeting vectors, i.e., vectors that are useful for causing directed mutation(s) in a target gene. More particularly, the preferred use of the gene targeting vectors of the present invention is for the mutation of genes in embryonic stem cells, wherein these mutated stem cells are then useful for the production of mutated whole animals, particularly mice.

### BACKGROUND OF THE INVENTION

The Human Genome Project, which began in 1991, is expected to provide about 90% complete human genome sequence coverage by the year 2001. See. *e.g.* Collins, *et al.,* Science,**282:**682-689, 1998. With this will be the discovery of tens of thousands of new genes with unknown function. As detailed below, inactivation of genes in the mouse genome by homologous recombination is one of the best, albeit not one of the fastest, ways to assess function of a novel gene. In order to address this new demand for deciphering gene function it will be important to develop methods to make gene targeted mice faster and more efficiently. Using conventional methods of making gene targeted mice, the majority of time is spent on building the targeting vector. Clearly, methods that allow a reduction in the time spent building gene targeting vectors will have a large impact on the total working hours invested in making gene targeted mice.

Mutations have long been a tool used to understand gene function. By observing the physical or biochemical defects that result when a gene is perturbed we can deduce, at least in part, the function of that gene. See. *e.g*. Castle and Allen, Proc. Am. Acad. Arts Sci.,**38**:603-621, 1903. Mutations can be found as spontaneous events in populations of organisms or induced with mutagens, such as the chemical mutagen N-ethyl-N-nitrosourea. Basic understanding of a mutation involves mapping it or grouping it into a "complementation group" in order to define what gene a given mutation belongs to, or whether the mutation defines a novel gene. The process of mapping and genetic complementation testing involves breeding experiments, typically over several generations of the organism in question. For this reason, simple organisms, such as the bacteria *Escherichia coli*, the yeast *Saccharomyces cerevisiae*, the round worm *Caenorhabditis elegans*, and the fruit fly *Drosophila melanogaster*, all with relatively short generation times, have been popular for experiments whereby novel mutations are induced through mutagenesis protocols and then characterized by genetic mapping and complementation testing. Until the late 1980's, mice, which are a popular organism for studying aspects of human physiology because of their relative physiological similarity, have not been as popular for studying genetics. Compared to the above-mentioned simple organisms, mice have a much longer generation time, and their size and husbandry requirements do not typically permit the rearing of the large numbers required in classical genetic experiments.

In late 1980's it became possible to create mutations in specific genes of interest in mice by the isolation of mouse embryonic stem cells (see. *e.g*. Martin, Proc. Natl. Acad. Sci. USA,**78:**634-638; 1981, Evans and Kaufman, Nature,**309:**154-156, 1981), and the discovery that the phenomenon of "homologous recombination" could occur in mammalian cells (see. *e.g.* Smithies, *et al.*, Nature,**317:**230-234, 1985). These combined discoveries quickly led to a procedure that allowed one to inactivate a specific gene by first inactivating that gene in embryonic stem cells, and then transferring embryonic stem cells bearing the desired mutation into developing mouse embryos. See. *e.g*. Doetschman, *et al.*, Nature,**301:**576-578, 1987; Thomas *et al.*, Cell,**219:**419-428, 1986. While this procedure is relatively laborious from the point of view of the work required to make a single mutation, it is efficient considering that a precisely defined mutation could be created in about 1 year using relatively few mice. Since these first demonstrations of targeted gene inactivation in mice, thousands of genes have been targeted and studied in this manner. The method has proven to be an invaluable tool for deciphering the complex functions of genes in the context of *in vivo* mouse biology. For example, the β-2-microglobulin gene was found to be necessary for the efficient development of the CD-4⁻,CD-8⁺ class of T-cells, and consequently for the efficient production of certain classes of antibodies to certain antigens. See. *e.g.* Koller, *et al.*, Science,**248:**1227-1230; 1990; Spriggs, *et al.*, Proc. Natl. Acad. Sci. USA,**89:**6070-6074, 1992. The connexin 43 gene, initially regarded as important in some of the very first cell differentiation events of mouse embryogenesis, was found to be most important in the developing heart. See. *e.g*. Reaume, *et al.*, Science,**216:**1831-1833, 1995. The JNK-3 gene was shown to be necessary in the cell death pathway in the brains of mice treated with the excitotoxin kanic acid. See. *e.g*. Yang, *et al.*, Nature,**389:**865-870, 1997.

Beyond just learning about the functions of genes, in some cases the mutant animals represent useful tools to study disease processes by allowing researchers to test potential therapeutic reagents. For example, U.S. Patent No. 5,777,195 describes a knockout of the mouse DARPP-32 gene and its utility as a screening model for potential therapeutic agents useful in the treatment of schizophrenia, Parkinson's disease, and the treatment of addictions, especially those involving drugs of abuse. U.S. Patent No. 5,814,300 describes gene-targeted heterozygous and homozygous SOD-1 null non-human mammals and their use for determining the effectiveness of a compound for counteracting the deleterious effects of oxidative stress. Another example may be found with U.S. Patent No. 5,859,307 which describes gene targeted mutants for the mouse RAG-1 gene and their potential use in the establishment of continuous lymphoid cell lines, as well as the establishment of animals developing tumors for use in studying tumor cell developments and treatments.

The method of making gene-targeted mutations in mice has proven particularly useful for the complex issue of deciphering the functions of individual family members in gene families. For example, there are 4 members of the prostaglandin E2 receptor family. Audoly, *et al.*, Am. J. Physiol.,**277:**H924-30, (1999) have shown that only the prostaglandin E2R EP1 member is responsible for perception of certain pain stimuli. Disruption of all 3 superoxide dismutase genes has shown that only Mn-superoxide dismutase is absolutely essential for survival. See. *e.g.* Carlsson, *et al.*, Proc. Natl. Acad. Sci,**295:**6264-6268, 1995; Reaume, *et al.*, Natur. Genet.,**13:**43-47, 1996; Li, *et al.*, Nature Genet.,**390:**376-381, 1995. Disruptions of all of the members of the src-kinase family have uncovered a diverse set of functions for each of the family members. See. *e.g*. Lowell and Soriano, Genes Dev.,**10:**1845-1857, 1996.

The method of homologous recombination allows one to also make subtle changes in a gene without completely disrupting its function. The creation of a series of mutant versions of the gene that still possess partial activity has proven to be a complementary tool to completely inactivating a gene. See. *e.g*. Nagy, *et al.*, Curr. Biol.,**8:**661-664, 1998. In some instances mutations associated with human genetic disorders do not inactivate the gene but rather alter its function or constitute a "gain of function". Homologous recombination strategies that create more subtle changes can be employed in order to recapitulate these sorts of mutations in mice. For example, Sullivan, *et al.*, J. Biol. Chem.,**272**:17972-17980, (1997) have replaced the mouse apolipoprotein E gene with all 3 versions (alleles) of the human gene in order to more faithfully replicate the physiological effects associated with each of these 3 alleles. U.S. Pat No. 5,777,194 describes mice in which the Aβ portion of the mouse APP gene has been replaced with the human version of the gene in order to more faithfully replicate the physiology of Aβ deposition in the human brain, while providing a more sensitive assay with which to track Aβ levels.

### SUMMARY OF THE INVENTION

This invention generally involves a process with which to create gene-targeting vectors in a rapid method by use of a specialized genomic DNA library, homologous recombination in yeast, and a subsequent selection system in *E. coli*. More particularly, this invention concerns a process with which to create gene-targeting vectors that may be used in mouse embryonic stem cells. The method involves using homologous recombination in yeast to simultaneously: 1) screen for genomic clones of interest and 2) build a gene targeting vector by virtue of replacing a defined portion of a gene of interest with a positive selection marker that can be used in both *E. coli* and mammalian cell culture.

In a first embodiment, the present invention provides a method of preparing a gene targeting vector, said method comprising transforming yeast cells with a RKO clone and a yeast targeting cassette (YTC), wherein said RKO clone comprises a genomic clone insert, a yeast replication element, a yeast selectable marker, a bacterial origin of replication, optionally a bacterial selectable marker, and optionally a mammalian negative selection marker, and wherein said YTC comprises a bacterial/mammalian positive selection marker flanked by recombinogenic arms, maintaining said yeast cells under conditions wherein said RKO clone and said YTC undergo homologous recombination via said genomic clone insert and said recombinogenic arms to produce a gene targeting vector, selecting transformed yeast cells by their expression of said yeast selectable marker on said gene targeting vector or on said RKO clone, isolating said gene targeting vector and said RKO clone from said selected yeast cells, transforming bacterial cells with said gene targeting vector and said RKO clone, selecting transformed bacterial cells that grow on selective media that is selective for bacterial cells expressing said bacterial/mammalian positive selection marker, thereby selecting for bacterial cells transformed with said gene targeting vector, and isolating said gene targeting vector from said selected bacterial cells.

In a second embodiment, the present invention provides a method of preparing gene targeted mammalian cells having a targeted gene mutation, said method comprising transforming mammalian cells with said gene targeting vector of the first embodiment of the invention, maintaining said mammalian cells under conditions wherein said gene targeting vector and the genome of said mammalian cells undergo homologous recombination to produce a gene targeted mammalian cell, and selecting gene targeted mammalian cells wherein homologous recombination has occurred by selecting gene targeted mammalian cells for their expression of said bacterial/mammalian positive selection marker, thereby obtaining gene targeted mammalian cells containing said targeted gene mutation.

In a third embodiment, the present invention provides a method of making gene targeted mice, said method comprising combining a gene targeted mouse cell according to the second embodiment of the invention with an early mouse embryo to produce a gene targeted embryonic construct, and introducing said gene targeted embryonic construct into a female host mouse, wherein said gene targeted embryonic construct is allowed to mature into a chimeric live whole mouse, said whole mouse thereby having a genome that includes said targeted gene mutation.

In a fourth embodiment, the present invention provides a gene targeting vector comprising a yeast replication element, a yeast selectable marker, a bacterial origin of replication, optionally a bacterial selectable marker, optionally a mammalian negative selection marker, and a genomic clone insert containing a bacterial mammalian positive selection marker inserted therein.

### DESCRIPTION OF THE FIGURE SHEETS

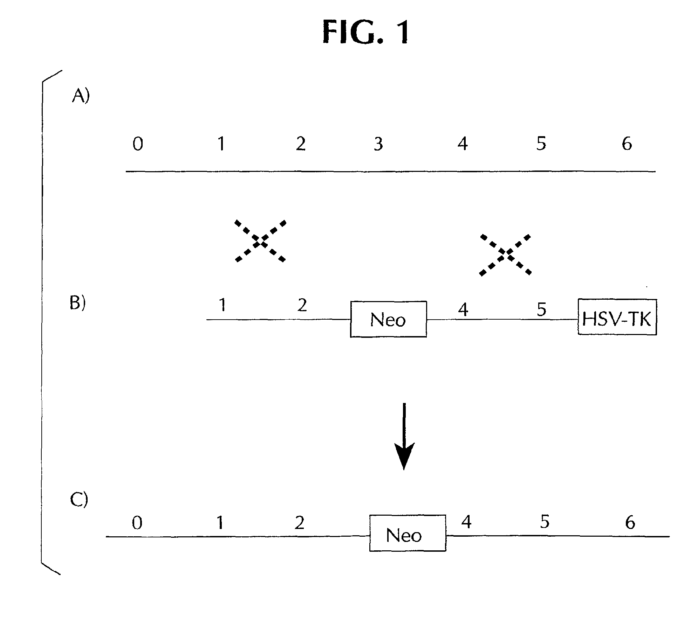

FIGURE 1 provides a general paradigm for introducing mutations into a mammalian genome using homologous recombination. In the figure, homologous recombination between elements A and B results in a product C wherein region 3 has been replaced by a marker segment (Neo). The negative selection marker (Hsv-TK) is separated from the positive selection marker in the process of homologous recombination.

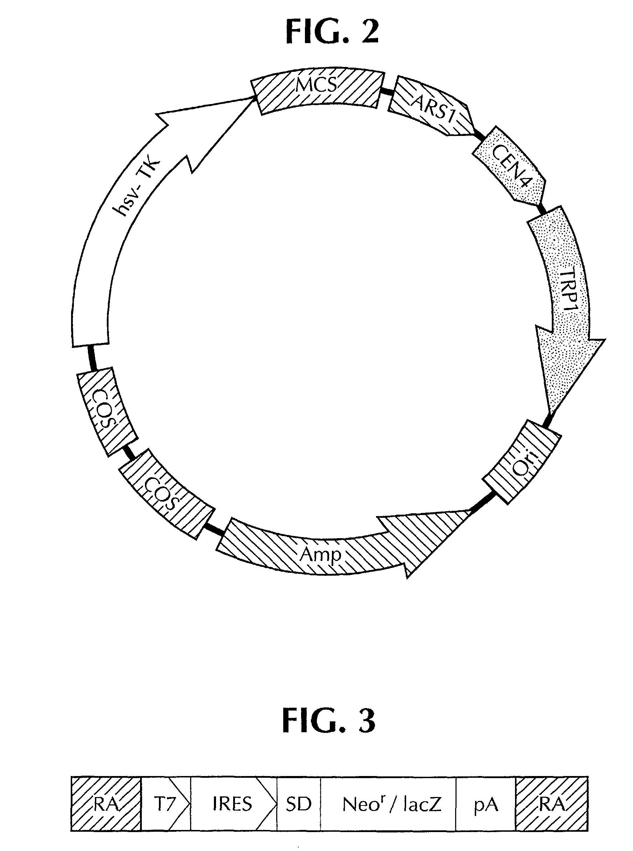

FIGURE 2 provides a plasmid map of a preferred RKO backbone according to the present invention. ARS1 is a yeast autonomous replicating sequence that serves as a yeast replication element. CEN4 is a yeast centromeric sequence that helps to stabilize propagation of the vector in yeast. TRP1 is a yeast selectable marker. Ori is a bacterial origin of replication. Amp is a bacterial selectable marker. The hsv-TK cassette is a mammalian negative selection marker. Addition of the genomic clone insert to this plasmid would yield an RKO clone. In a preferred embodiment, genomic clone inserts would be inserted into the multiple cloning site (MCS) shown in this plasmid to create the RKO clone. Addition of Cos sites allow the RKO clone to be packaged in lambda phage as a cosmid clone for propagation purposes.

FIGURE 3 depicts an example of a preferred yeast-targeting cassette (YTC) as described in this invention. The bacterial/mammalian selection marker cassette is flanked by recombinogenic arms (RA). A promoter (T7) allows transcription of the bacterial/mammalian positive selection marker (Neo^{r}) to occur in bacteria. An internal ribosome entry site (IRES) element allows translational initiation of the said selection marker to occur when the said marker is transcribed by a promoter in the genomic region of interest (GRI). A Shine-Dalgarno element (SD) allows translation of the said selection marker to occur in bacteria. Fusion of the coding sequence for the said selection marker with the coding sequence for a reporter protein such as bacterial beta galactosidase (lacZ) creates a reporter system for the GRI so that it may be easily possible to determine when a gene of interest is being expressed. A polyadenylation signal (pA) is desirable if transcription in mammalian cells initiates from the (GRI).

### DETAILED DESCRIPTION OF THE INVENTION

### Definitions

**Arms of Homology:** A pair of DNA segments that is present in the gene targeting vector; these segments are homologous to two portions of the target gene. Because of this homology, the arms of homology will be able to undergo homologous recombination with the target gene. The arms of homology are preferably at least 200 bp in length to allow for appreciable rates of homologous recombination to occur in mammalian cells. More preferably, the arms of homology will be at least 2 kb or greater in length.

**Bacterial Origin of Replication:** A genetic element that enables replication of the DNA sequence of which it is a part during each cell cycle in a bacterium, such that the DNA sequence will be maintained in each daughter cell. Examples include pMB1, pSC101 and pUC.

**Bacterial Selectable Marker:** A genetic element encoding a protein that when expressed in bacteria enables selection of the bacteria by the presence of the protein. Thus, any bacteria that contain and express a bacterial selectable marker can be differentiated from otherwise similar bacteria that do not contain and express the marker. Examples include Amp^{r}, Tet^{r}, and Kan^{r}.

**Bacterial/Mammalian Positive Selection Marker:** A genetic element encoding a protein that when expressed in a bacterial or mammalian cell enables selection of the cell for the presence of the protein. In an appropriate selective environment, only cells expressing the marker protein should survive and multiply. Thus, any cell that contains and expresses a bacterial/mammalian positive selection marker can be differentiated from otherwise similar cells that do not contain and express the marker. Examples include Neo^{r}, Zeo^{r} and *bsd.*

**Early Mouse Embryo:** A mouse embryo that is capable of being combined with a gene targeted mouse cell as defined herein and developing into a chimeric adult mouse. Preferably, an early mouse embryo has developed 2.5 days *post-coitus* (dpc) to 3.5 dpc, which is approximately equivalent to the 8-cell stage through the blastocyst stage in development.

**Embryonic Stem Cells:** Stem cells of embryonic origin. Preferably, embryonic stem cells are derived from embryos that have developed to 3.5 dpc, which is approximately equivalent to the blastocyst stage in development.

**Gene Targeted Cell:** A cell containing a non-naturally occurring genomic mutation as a result of homologous recombination between the cell genome and a gene targeting vector as defined herein.

**Gene Targeted Embryonic Construct:** An embryo resulting from the combination of an early mouse embryo with a gene targeted cell. When introduced into a female host mouse, a gene targeted embryonic construct is capable of developing into a chimeric live whole mouse.

**Gene Targeting Vector:** A DNA molecule that results from homologous recombination between a RKO clone and a YTC as defined herein. A gene-targeting vector contains a mutated target gene that is ready to knockout or otherwise mutate its counterpart gene via homologous recombination when inserted into a cell containing that counterpart gene.

**Genomic Clone Insert:** A DNA segment that originates from the genome of a particular organism of interest. A genomic clone insert is part of a RKO clone as defined herein. Typically, this segment is derived from the creation of a library of clones from a genome. One aspect of the present invention is the selection of a specific desired genomic clone insert during the process of creating the gene-targeting vector. However, at the beginning of the invention method, RKO clones are produced that contain numerous genomic clone inserts, not only the desired one, and thus this term encompasses all such DNA segments derived from the genomic library.

**Genomic Region of Interest (GRI)** A contiguous sequence of genomic DNA comprising greater than 100 base pairs and which one wants to specifically alter using the present invention. Typically the GRI will include the coding portion of a gene of interest but could also include a regulatory region that one wants to alter in a specific way. Still another example involves an intervening sequence between a gene(s) of interest that one may want to alter in some way in a multistep process, part of which involves creating specific alterations using the present invention.

**Homologous Sequence:** A sequence that is at least about 90% identical to the comparison sequence, and is preferably greater than 95% identical. Sequence identity, as used throughout the present application and claims, is defined using the BLAST software for making sequence comparisons.

**Mammalian Negative Selection Marker:** A genetic element that when present in a mammalian cell enables selection of the mammalian cell for the absence of the element. In an appropriate selective environment, any cells expressing the marker protein will not survive and/or multiply. Thus, any mammalian cell that contains a mammalian negative selection marker can be differentiated from otherwise similar cells that do not contain and express the marker. Examples include Herpes Simplex Virus-Thymidine Kinase (HSV-TK), Diptheria toxin (DT), and a promoter that makes antisense RNA designed to inactivate a positive selection marker (Skryabin and Schmauss, Transgenic Res.,**6:**27-35, 1997). Examples of negative selection markers useful in the present invention are described in U.S. Patent No. 5,464,764.

**Multiple Cloning Site:** A DNA segment that is optionally present in the RKO clone; this segment comprises a series of very closely situated restriction enzyme recognition and cutting sites. In fact, the sites are frequently adjacent and possibly even overlapping.

**Mutation:** A change in the sequence of a DNA segment, such that the sequence is no longer identical to a naturally occurring sequence.

**Recombinogenic Arms (RAs):** A pair of DNA segments that are present in the YTC; these segments are homologous to two portions of a target gene. Because of this homology, the recombinogenic arms will be able to undergo homologous recombination with the target gene. The RAs are preferably at least 20 bp in length to allow for appreciable rates of homologous recombination to occur in yeast. More preferably, the RAs will be at least 100 bp in length.

**RKO Backbone:** This is the RKO clone without the genomic clone insert. Because this vector is grown in bacterial cells to create and manipulate it, a bacterial selectable marker is typically present, and therefore still present in the RKO clone. However, the presence of this marker is unnecessary for practicing the methods of the present invention.

**RKO Clone:** A DNA molecule comprising a genomic clone insert, a yeast replication element, a yeast selectable marker, a bacterial origin of replication, optionally a bacterial selectable marker, and optionally a mammalian negative selection marker, each as defined herein. The RKO clone may include additional genetic elements as well. RKO is an acronym for Rapid KnockOut, which is the terminology used to describe the preferred use of the present invention, for rapidly building a gene-targeting vector. . Homologous recombination in yeast between a YTC and an RKO clone yields a gene-targeting vector.

**Stem Cells:** Pluripotent or totipotent cells that are capable of being combined with an early embryo and subsequently maturing into a chimeric adult.

**Substantially Isogenic:** A sequence that is at least about 97-98% identical to the comparison sequence, and preferably is at least about 99% identical.

**Targeted Gene Mutation:** A mutation created in the GRI by practice of the present invention. The mutation is created by virtue of homologous recombination occurring between the RAs(recombinogenic arms) of the YTC and the genomic clone insert of the RKO clone so as to create a gene-targeting vector. The targeted gene mutation is subsequently transferred into the genome of the target cell and/or organism by homologous recombination between the gene-targeting vector and the endogenous GRI within said target cell.

**Yeast Replication Element:** A genetic element that enables replication of the DNA sequence of which it is a part during each cell cycle in yeast, such that the DNA sequence will be maintained in each daughter cell. Examples include 2µ origin of replication, a yeast autonomous replicating sequence (ARS) including those sequences which may be found in the genomes of other organisms that allow replication in yeast (Larionov, *et al.*, Proc. Natl. Acad. Sci. USA,**93:**491-496, 1996).

**Yeast Selectable Marker:** A genetic element encoding a protein that when expressed in yeast that enables selection of the yeast cell by the presence of the protein. Thus, any yeast cell that contains and expresses a yeast selectable marker can be differentiated from otherwise similar yeast cells that do not contain and express the marker. Examples include TRP1, HIS3, URA3, and LEU2.

**Yeast Targeting Construct (YTC)**: A DNA molecule comprising a bacterial/mammalian positive selection marker flanked by recombinogenic arms, each as defined herein. The YTC may comprise additional genetic elements as well. In a preferred embodiment of this invention the bacterial/mammalian positive selection marker is constructed in such a way that, while capable of being expressed in bacteria under all normal growth conditions, it is only expressed in mammalian cells if it is inserted within a gene of the mammalian cell. For example, if the bacterial/mammalian positive selection marker has no mammalian promoter but has a bacterial promoter and an internal ribosomal entry site (IRES) element, then mammalian positive selection will only occur if the bacterial/mammalian positive selection marker is inserted within a gene that is being expressed. Alternatively, if the bacterial/mammalian positive selection marker lacks a polyadenylation signal then again mammalian positive selection will only occur if the marker is inserted within a gene that has a polyadenylation signal (as should be the case in most gene-targeting experiments).

Figure 1 depicts the general paradigm for introducing mutations into a mammalian genome using homologous recombination (reviewed in Capecchi, Trends Genet, **217:**70-76, 1989; and Koller and Smithies, Ann. Rev. Immunol, **218:**705-30, 1992). This general paradigm is applicable to the methods of the present invention, as well as to prior art methods. A length of genomic DNA is first depicted by organizing it into regions (numbered 0-6 in FIG. 1, element A). In FIG. 1, region 3 is designated to be deleted. Homologous recombination using a gene-targeting vector is utilized. The type of gene-targeting vector used for the deletion of a gene is termed a replacement or deletion vector. "Deletion vector" as used herein refers to a vector that includes one or more selectable marker sequences and two sequences of DNA homologous to the genomic DNA that flank the DNA gene sequence to be deleted. These flanking sequences are termed arms of homology. In FIG. 1, element B, the arms of homology are represented by regions 1-2 and 4-5. Preferably, these arms of homology are substantially isogenic for the corresponding flanking sequences in the cell being targeted or "target cell." The use of DNA that is substantially isogenic to the target cells DNA helps assure high efficiency of recombination with the target sequences (See U.S. Pat. No. 5,789,215). The deletion vector preferably includes at least a positive selection marker within the arms of homology to enable the scoring of recombination. Such positive selection markers can confer a phenotype not normally exhibited by wild-type mammals; for example, resistance to a substance normally toxic to the target cells. In another example, the deletion vector also includes one or more negative selection markers outside the arms of homology to facilitate identification of proper homologous recombinants. U.S. Patent No. 5,464,764 describes the use of such "positive-negative" selection methods. The negative selection markers can confer, for example, sensitivity to a substance not normally toxic to the target cell. The selection markers can be gene cassettes, i.e., include both a promoter and an accompanying coding sequence. The result of homologous recombination of the gene-targeting vector with cellular DNA in the paradigm is shown in FIG. 1, product C. As depicted therein, region 3 has been replaced by the positive selection marker. Upon successful gene-targeting and homologous recombination, the positive selection marker is incorporated into the genome within the arms of homology in place of the deleted gene segment, while the negative selection marker is excluded. Thus, to enrich for homologous recombinants, gene-targeted cells are grown in culture medium containing the appropriate positive and negative selective compounds.

The "target cells" are those cells to be mutagenized. The target cells preferred for use in the present invention are mouse embryonic stem cells. However, other cells can be utilized. If embryonic stem cells are to be targeted then one can utilize properly identified gene targeted cells to combine with early mouse embryos to create a gene targeted embryonic construct. Preferred methods for combining gene-targeted cells with early embryos include blastocyst injection and aggregation methods. The gene targeted embryonic construct is then transferred to a host animal, preferably a pseudopregnant female mouse. Some of the resulting offspring mice will mature and produce gametes that bear the desired mutation. These particular mice are referred to as "germline chimeras". Some of the offspring of germline chimeras bear one mutant copy of the gene of interest in all of the cells in their body and are referred to as "heterozygotes". If two of these heterozygotes are mated to each other some of their offspring will bear two mutant copies of the gene of interest and are referred to as homozygotes.

The method described herein uses a genomic DNA library made in a vector with the following features:
a yeast replication element;
a yeast selectable marker;
a bacterial origin of replication;
optionally a bacterial selectable marker; and
optionally a negative selection marker
to which is added a genomic clone insert. An example of such a vector is shown in figure 2.

If the library is to be a cosmid library then at least one Cos site must be included. In a preferred embodiment, the vector includes two Cos sites in the same orientation in tandem repeat with a unique restriction site separating the two. For a thorough description on the use of Cos sites to build cosmid libraries see Maniatis, *et al.*, Cold Spring Harbor, New York, (1982), incorporated herein by reference.

In another preferred embodiment, a region adjacent to the negative selection marker is a multiple cloning site containing a large number of rare cutters (e.g., 6 or more base pairs in the recognition sites). Preferably these sites are strategically placed. For example a *BamH*I site, which is often used as a recipient site to receive genomic DNA that has been partially digested with the enzyme *Sau*3A (see Maniatis, *et al., supra*), would preferably be placed in a center position relative to the total collection of enzyme sites in this region.

A genomic DNA library may be constructed making a RKO clone with the features described above. For the purposes of making gene-targeting vectors in mammalian cells, the source of DNA is preferably from an isogenic source. Such would be the case if mouse cells were to be targeted and genomic DNA used to make the gene targeting vector was isolated from the same strain of mice. Further description on the use of isogenic DNA can be found in U.S. Pat. No. 5,789,215. Methods used to make genomic DNA libraries are described in Maniatis *et al., supra.*

A preferred embodiment of this invention involves screening a genomic library comprising the above features by combining a DNA library (an aliquot of DNA representing either the entire library of recombinant genomic clones or a subset of the library) with a fragment of DNA called the yeast targeting cassette (YTC). A DNA aliquot representing the entire library can be obtained by inoculating a culture with a number of colony forming units or plaque forming units equal to or greater than the total number of independent genomic clones known to be in the library. An aliquot of DNA appropriately prepared from this culture would then represent the entire genomic library. Similarly if the culture was initially inoculated with fewer clones than what represented the entire library then DNA prepared from this culture would represent a subset of the entire library.

A preferred method of screening the library involves dividing the library into pools of recombinant clones representing subsets of the library. All of the pools are screened by either conventional polymerase chain reaction (PCR) methods designed to identify a GRI or by hybridizing radioactively labeled probes to samples of the pools that have been blotted onto a suitable membrane. Clones bearing the GRI are then further isolated by mixing them with the YTC and performing the procedures described herein.

An example of a preferred YTC is shown in figure 3. The YTC comprises a bacterial/mammalian positive selection marker flanked by regions of sequence identity to the GRI. These regions of sequence identity will serve as recombinogenic arms. The bacterial/mammalian positive selection marker can be flanked by loxP or FRT sites, thereby allowing for excision of the bacterial/mammalian positive selection marker (see, for example, U.S. Pat. No. 4,959,317). In a preferred embodiment of this invention the bacterial/mammalian positive selection marker is constructed in such a way that, while capable of being expressed in bacteria under all normal growth conditions, it is only expressed in mammalian cells if it is inserted within a gene of the mammalian cell. For example, if the bacterial/mammalian positive selection marker has no mammalian promoter but has a bacterial promoter and an internal ribosomal entry site (IRES) element, then mammalian positive selection will only occur if the bacterial/mammalian positive selection marker is inserted within a gene that is being expressed. Alternatively, if the bacterial/mammalian positive selection marker lacks a polyadenylation signal then again mammalian positive selection will only occur if the marker is inserted within a gene that has a polyadenylation signal (as should be the case in most gene targeting experiments).

In a further preferred embodiment of this invention the YTC will bear a reporter system capable of being expressed when inserted within the GRI. For example, if the bacterial/mammalian positive selection marker is to be expressed in mammalian cells by the GRI by virtue of an IRES element then the bacterial/mammalian positive selection marker may be a fusion protein between a reporter protein (such as betagalactosidase) and a selection protein (such as neo'). Alternatively, if the bacterial/mammalian positive selection marker is not expressed by the GRI, then the reporter protein can be expressed by the GRI by virtue of an IRES element that allows reporter protein translation to occur from mRNA transcripts driven by the native promoter in the GRI.

A preferred embodiment of this invention involves creating the YTC by use of a single PCR reaction using 2 chimeric oligonucleotides. The 3' portion of each oligonucleotide bears sequence identity to the selected mammalian positive selection marker. The 5' portion of each oligonucleotide bears sequence identity to the GRI. A conventional PCR reaction is performed using these two oligonucleotides and a plasmid bearing the bacterial/mammalian positive selection marker as a template. The resulting amplified product will be a bacterial/mammalian positive selection marker flanked by RAs, thereby forming the YTC.

The RAs are meant to allow homologous recombination to occur in yeast between genomic clones bearing the GRI (i.e., a RKO clone) and the YTC. Figure 2 illustrates aspects of the YTC and its regions of sequence identity with the GRI. Both RAs should be derived from the same chromosomal region of the GRI and must be oriented in the same direction and order as they are positioned in their endogenous chromosomal location. See e.g. WO 93/03183. They may either represent contiguous elements in the genome from which they are derived or be spaced by a gap. Preferably any gap is less than 2 million base pairs in size.

Following the step in which the YTC is mixed with library DNA, the mixture is transfected into a yeast strain bearing a mutation in a gene that is complemented by the yeast selectable marker. The transfected yeast are grown on minimal medium agar plates (selective conditions). All of the colonies that grow on these plates represent individually transfected yeast cells. Although not selected-for at this stage, yeast colonies that bear a genomic clone of interest and were also transfected by the YTC are susceptible to undergoing homologous recombination between the GRI and the YTC by virtue of the RAs flanking the YTC. Figure 1 illustrates the process of homologous recombination between the YTC and the GRI. Note that only yeast cells receiving a genomic clone bearing the GRI will undergo homologous recombination with the YTC and thereby incorporate the positive selection marker associated with the YTC. Also note that if the RAs on the YTC represent contiguous elements from the gene of interest then no deletion within the GRI occurs. However, if the regions of sequence identity represent noncontiguous elements from the GRI then the result of homologous recombination in yeast is a deletion of the entire region between the corresponding RAs. This latter strategy is useful for inactivating a gene of interest.

Depending on the complexity of the library aliquot that was used in the previous step, an appropriate number of yeast colonies are scraped off the agar plates. For example, if the library aliquot used represented 1,000 different genomic clones then 500 times this number, or 500,000 yeast clones, would preferably be scraped off of the agar plates. If the library aliquot represented 100 genomic clones, then 500 times this number, or 50,000 yeast clones, would preferably be scraped off of the agar plates.

The yeast clones that were scraped off would then preferably be subjected to one of several methods available to shuttle plasmids from yeast to bacteria such as the "smash and grab" procedure or Zymoprep™ (Zymo Research, Orange, CA) method. Briefly, the former method involves sonicating or vortexing the yeast cells in the presence of glass beads and phenol in order to crudely extract plasmid DNA. The crude DNA extract is then transfected into *E. coli* by conventional bacterial transformation protocols.

The transfected *E. coli* are then plated onto nutrient rich agar plates containing a drug that selects for the positive selection marker present in the YTC. Only genomic clones that bear the GRI and underwent homologous recombination with the YTC in yeast should bear a stable copy of the marker present in the YTC.

In this way genomic clones bearing the region of interest are isolated and simultaneously manipulated in order create a gene-targeting vector for mammalian cells. Note that the product that is created by homologous recombination in yeast bears two arms of homology in the correct orientation, separated by a positive selection marker and flanked on one side by a negative selection marker. Note that the relatively small recombinogenic arms of the YTC have essentially been increased in size by merging with the larger genomic clone insert of the RKO clone, thereby creating arms of homology, which are typically much larger than recombinogenic arms. RAs can be relatively small, as shorter segments will recombine in yeast at acceptable rates, whereas relatively longer homologous sequences are required for homologous recombination to occur at acceptable rates in mammalian cells.

Once the gene-targeting vector has been created, it is then preferably used to prepare gene targeted mammalian cells. Preferably, these cells are mouse cells, and can later be used to create a mouse carrying a targeted gene mutation.

First, mammalian cells are transformed with the gene-targeting vector by any method. There are numerous methods known in the art, and many more will likely be developed. Which method is used is not deemed critical to the practice of the present invention. After transformation, the cells are maintained under conditions that will allow gene-targeting vector to undergo homologous recombination with the GRI, thereby producing a gene targeted mammalian cell. Cells wherein homologous recombination has occurred may be selected by their expression of the bacterial/mammalian positive selection marker, which will have been inserted into the genome by the recombination event. Additionally, if the gene targeting vector included a mammalian negative selection marker outside the arms of homology, then the transformed mammalian cells wherein homologous recombination have occurred may be selected by their non-expression of this negative marker.

It is preferred that the mammalian cells used be stem cells. Even more preferred is the use of embryonic stem cells. However, any cell in which it would be desirable to create a targeted gene mutation may be used.

To make a gene targeted mouse, the gene-targeted cell as described above is used to grow a new whole live mouse. Preferably, this is done by taking the gene targeted mouse stem cell and combining it with an early mouse embryo to produce a gene targeted embryonic construct. What you will then have is an early mouse embryo that includes at least one cell carrying the targeted gene mutation as desired. Next, the gene targeted embryonic construct is introduced into a female host mouse, preferably a pseudo-pregnant female host mouse. The gene targeted embryonic construct will then be able to implant itself in the host mouse uterus, and mature in normal fashion into a live baby mouse. This baby mouse will be chimeric, with some cells derived from the early mouse embryo cells, and others derived from the gene targeted mouse stem cell. When this mouse is bred to other regular mice, some of their offspring will be heterozygotes whose genome includes the gene-targeted mutation.

It is frequently desirable to produce mice that are homozygous for the gene-targeted mutation. This is done by merely crossbreeding heterozygotes until a homozygote offspring is born. In some cases, the mutation will be lethal, and it will not be possible to obtain live homozygous mice. In other cases, it will be possible but extremely difficult.

The present invention is described in more detail with reference to the following non-limiting examples.

### EXAMPLES

### EXAMPLE 1 - Building of pCosRKO Cosmid Library Backbone Vector

The CEN4, ARS1, TRP1, Amp^{r}, and pUC ori elements of YCplac22 (Gietz and Sugino, Gene,**74:**527-534, 1988; obtained from American Type Culture Collection, 10801 University Boulevard, Manassas, VA) were amplified by polymerase chain reaction (PCR) (Mullis and F.A., Methods Enzymol.,**155:**335-350, 1987) using primers 44413-12A(CGC TTC GAA ATC GCT GGG CCA TTC TCA TGA; SEQ ID NO:1) and 44413-12B (CGC ATT TAA ATT CTT CCG CTT CCT CGC TCA CT; SEQ ID NO:2) which hybridize to the 5' end of the CEN4 domain and the 3' end of the pUC ori respectively. This PCR product was digested with the restriction endonucleases *BstB*I and *Swa* I to create sticky ends. Similarly the 2 Cos sites of plasmid SuperCos (Stratagene Inc., La Jolla, Calif.) were amplified using primers 44413-12C (GAC CGC TTA TCA TCG ATA AGC; SEQ ID NO:3) and 44413-12D (CGC ATT TAA ATG AAG GCT CTC AAG GGC ATC GG; SEQ ID NO:4). This PCR product was digested with the restriction endonucleases *Swa*l and *Cla*I to create sticky ends. The two digested PCR products were purified using agarose gel electrophoresis and ligated using T4 ligase. The ligation mixture was used to transform competent *E. coli* cells, and clones were identified that represented the correct product carrying the appropriate fragment of YCplac22 fused to the 2 Cos sites from pSuperCos. This construct was called YCpCos.

YCpCos was PCR amplified with primers 44413-12E (CGC GGA TCC TAG CCT GGC CAT CCG GGC CAT CGC TGG GCC ATT CTC ATG A; SEQ ID NO:5) and 44413-12F (CGC ATC GAT TTC GAT AAG CTC TGC TTT TTG T; SEQ ID NO:6). This PCR product was digested with the restriction endonucleases *BamH*I and *Cla* I to create sticky ends. Similarly, the herpes simplex virus thymidine kinase (hsvTK) expression cassette was amplified from the plasmid pJNS using the primers 44413-12G (CGC TTC GAA CTA CCG GGT AGG GGA GGC GCT; SEQ ID NO:7) and 44413H (CGC AGA TCT TCC GAT GGC CGC AGC GGC CTC TGA TGG AAT TAG AAC TTG GCA; SEQ ID NO:8). This PCR product was digested with the restriction endonucleases *BstB*I and *Bgl*II to create sticky ends. The two digested PCR products were purified using agarose gel electrophoresis and ligated using T4 ligase. The ligation mixture was used to transform competent *E. coli* cells, and clones were identified that represented the correct product carrying the hsvTK cassette fused into YCpCos. The construct was called YCpCosTK.

Finally, the multiple cloning site (MCS; GGCCGCTGCTTCCAGGCTAACACTTATATAGGCATTGGTACTGAGATACAGCG AGATACCGTGTAACTATAACGGTCCTAAGGTAGCGAAGGCTAACACTTATATA GGCATTGGTACTGAGATACAGCGAGATACCGTGCGGCCGCTGCGGCCAATTA ACCCTCACTAAAGGGAGGCCTATGCATACTAGTCCATGGGATATCCCCGGGG TTAACCCGCGGCTCGAGATCGATGAATTCGGCCGGCCGTTTAAACGGATCCG GCGCGCCTTAATTAAAAGCTTCATATGTCTAGAGTCGACGAGCTCGGTACCGG GCCCGCATGCTGGCCAAGGCCTAGATCTCCCTATAGTGAGTCGTATTATGGC AAACAGCTATTATGGGTATTATGGGTAGGCCATCCGGGCCGCGGCCGCTTCC ATCCGGGCC; SEQ ID NO:9) was created using a series of overlapping and complementing oligonucleotides and cloned into YCpCos by linearizing YCpCos using the restriction endonuclease *Sfi*l. The resulting plasmid was called pCosRKO.

### EXAMPLE 2 - Building a Genomic Library with pCosRKO

Mouse genomic DNA was isolated from one kidney of a female DBA/1lacJ mouse using a standard proteinase K digestion followed by phenol/chloroform extraction and ethanol spooling of the DNA. Briefly, the kidney was bisected approxiamately 10-15 times with sterile scissors. The minced kidney was incubated in 5 ml of a tissue digestion buffer (50mM Tris Cl (pH 8.0), 100 mM EDTA (pH 8.0), 100mM NaCl, 1% SDS, 600 µg/ml proteinase K (Roche, Cat. No. 1 373 196)) overnight at 55°C. The digestion was then extracted with an equal volume of a 1:1 phenol/chloroform mixture. DNA was then precipitated by adding two volumes of ethanol and spooling the DNA using a sterile glass rod. Finally, spooled DNA was scraped off of the glass rod into a 1.5 ml tube and resuspended in 300 µl of TE (10 mM Tris Cl (pH 7.5) 1mM EDTA). Quality of the isolated DNA was checked by running uncut DNA on a pulse field agarose gel apparatus along with molecular weight markers (Low Range PFG Marker; New England Biolabs® Inc., Beverly, MA). Specifically, a CHEF-DR® II apparatus(BioRAD, Hercules, CA) was used with a 1% agarose gel, 1-6 second switch time, ramped over 11 hours, and 6 V/cm at 120° angle. The gel was run for 11 hours. Good quality DNA was at least 150 kb or larger. A series of partial digests of the genomic DNA with the enzyme *Sau*3A were performed by mixing 15 µl of DNA with 1.5 units of enzyme (Boerhringer Manheim) in a 100 µl reaction volume. The mixture was preheated to 37°C before adding enzyme. 15 µl aliquots were removed at 0, 1, 2, 3, 4, 8, and 15 minutes after adding enzyme. Immediately after removing an aliquot 3 µl of 0.5M EDTA was added to the aliquot and the mixture was placed at 65°C for 10 minutes. The aliquots were loaded onto a pulse field gel apparatus and run as described above. It was determined that a 2.5 minute digest time was ideal as it generated a mean fragment length that was closest to the preferred 30 - 40 kb length. A large scale *Sau*3A digest was performed by mixing 150 µl of DNA with 15 units of enzyme in a 1000 µl reaction volume. Again, the mixture was preheated before the enzyme was added. The 1 ml reaction was loaded onto a 17 ml 40%-10% sucrose gradient (see Maniatis *et al., infra*) and spun at 26K rpm for 24 hours at 15°C. Every third aliquot was ethanol precipitated, and resuspended in 50 µl of TE (10 mM Tris (pH 7.5), 1 mM EDTA). 10 µl of each TE-resuspended aliquot was run on a pulse field gel with markers as described above. From this analysis it was inferred which aliquots contained mostly fragments that were between 30 and 40 kb. The 30-40 kb-prevalent aliquots were pooled.

PCosRKO vector DNA was prepared by digestion with *Pvu*II, treatment with calf intestinal phosphatase, and then digestion with *Bam*HI. Individual vector arms were isolated on a 0.7% agarose TBE gel using Qiagen® gel extraction kit (Qiagen, Valencia, CA). One µg of the double digested pCosRKO vector was ligated to 4.0 µg of the size selected *Sau*3A genomic DNA in a 10 µl reaction volume. Ligation was performed at 4°C overnight. Then, the ligation reaction 1 µl of the ligation mixture was packaged using Gigapack Gold packaging extract (Stratagene, LaJolla, CA).

Following the 2 hour packaging reaction, the titer of the library was determined by diluting the packaging reaction 1:10, 1:50, and 1:100 in SM buffer, adsorbing phage to properly prepared XL-1 Blue MRA cells (Stratagene, La Jolla, CA), and plating on LB-ampicillin plates as described by the packaging extract manufacturer (Stratagene, La Jolla, CA). Numbers of colonies from each of the dilutions was determined. A titer is calculated by multiplying the number of colonies produced for a given dilution by the dilution factor and is expressed as colony forming units (cfu) per µl.

The library was plated in DNA format by inoculating 1000 cfu into 7 ml of semisolid LB-agar and grown for 48 hours at 30°C in 8-hole tubes (Autogen , Framingham, MA; cat no. AGP3210-10022) as described in the GeneTrapper™ protocol for semi-solid amplification of plasmid cDNA libraries (Life Technologies, Bethesda, MD). Colonies were harvested from the semisolid agar by centrifuging at 3000 rpm for 30 minutes. The agar was decanted and a conventional cosmid preparation procedure performed on an Autogen 740 robot (Autogen, Framingham, MA).

One hundered and twenty 8-hole tubes were inoculated in this way and ten 96-well DNA plates were generated. The ten DNA plates were ordered into a 2 x 5 matrix forming a matrix of 24 wells x 40 wells. 2 µl from the well of every row of this 24 x 40 matrix were removed and pooled into 40 "row pools". Similarly 2 µl from the well of every column were removed and pooled into 24 "column pools". Finally, 2 µl from the well of every plate were removed and pooled into 10 "plate pools". In this way each well was represented in a "row pool", a "column pool", and a "plate pool". TE was added to each pool to bring the total volume to 150 µl.

### EXAMPLE 3 - Screening a pCosRKO Genomic Library

### A) PCR-Screening Cosmid Pools

In order to screen for a GRI, at least a partial sequence for that gene should be known. From the available sequence information, PCR primers are designed to amplify a region of genomic DNA, preferably about 300 bp in size. A 1 µl aliquot from each of the 74 row, column, and plate pools described above is used to PCR screen the pools in 96-well plate format using a conventional PCR amplification with a Perkin Elmer GeneAmp® PCR System 9600 (Applied Biosystems Inc., Norwalk, CT). Positive row, column, and plate pools identified by this method are used to identify which of the 960 pools in the entire library of pools contain genomic clones of interest.

### B) YTC amplification

Concurrent with library screening a YTC can be generated by PCR. A YTC is created by PCR amplification of a bacterial/mammalian positive selection marker using chimeric oligonucleotides that also bear identity to the gene of interest. Bacterial/mammalian positive selection markers that are routinely used include the neomycin neo^{r}-cassette driven by dual bacterial/mammalian promoter in the plasmids pDual (Stratagene, La Jolla, CA) and a modified variant of pDual in which a NotI site has been inserted between the promoters and the neo^{r} coding sequence (pDual-NotI). The primers 44413-29FA (CCA TCA ACA CGC GTC TGC GTT CGA CCA GGC TGC GCG TTC TCG CGG CCA TAG CAA CCG ACG TAC GGC GTT GCG CCC TCG CCG GCA GCA AGA AGC CAC GGA AGT CAT GTG CGC GGA ACC CCT ATT TGT T; SEQ ID NO:10) and 44413-29RA(GAG CCA GAA CGG CGT CGG TCA CGG CAT AAG GCA TGC CCA TTG TTA TCT GGG CGC TTG TCA TTA CCA CCG CCG CGT CCC CGG CCG ATA TCT CAC CCT GGT CGA GAT AGC GCG GGT TCC TTC CGG TAT; SEQ ID NO:11) are used to amplify a neo^{r}-cassette from pDual-Notl. The 3' 23 base pairs of each of these primers bears identity to the 5' and 3' border of the pDual-Notl neo^{r}-cassette, respectively, while the 5' 103 base pairs of each oligonucleotide bear identity to regions in the hsvTK cassette of pCosRKO that we will call RA1 and RA2. Following PCR amplification the 1.6 kb product is gel isolated by running the PCR reaction out on a 0.8% TBE agarose gel and extracting the band using a Qiagen® gel extraction kit (Qiagen, Valencia, CA).

### C) Yeast co-transfection of cosmid clone with YTC

For the purposes of an experimental control, an analogue of pCosRKO was made such that the regions RA1 and RA2 were deleted by cutting pCosRKO with the enzymes *Age*I and *Bsp*EI, "polishing" the ends with T4 DNA polymerase, and religating the plasmid. This control plasmid was called pCosRKOdelta.

pCosRKOdelta plasmid DNA was mixed with pCosRKO plasmid DNA in molar ratios of 1:1, 1:10, 1:100, and 1:1000. For each yeast transfection 100 ng of DNA from one of the above mixtures was used. To prepare a DNA mixutre for transfection, DNA was linearized with the enzyme I-Ceul that cuts between two 50 base pair repeats in order to enhance transfection efficiency as described by Raymond et al. (BioTechniques, **27:**892-96 (1999)). The DGY63 yeast strain (R.D. Geitz, University of Manitoba) was transfected with 100 ng of I-Ceul digested pCosRKO and either 160 ng of the hsvTK-YTC described above according to the TRAFO method described (Agatep et al., Technical Tips Online, (http://tto.trends.com) (1998)) with the exception that all solutions and media were made with ES Cell Qualified Ultra Pure H₂O (Specialty Media, Phillipsburg, NJ; cat # TMS-006-A). From the final suspension of transformed yeast in this protocol, 0.5 µl is diluted to 100 µl with sterile water and plated onto a 100 mm SC-Trp plate (Agatep et al., Technical Tips Online, (http://tto.trends.com) (1998)). In addition 5µl , 50 µl, and 500 µl were diluted to 3 ml and plated onto 500 cm² SC-Trp plates (Agatep et al., Technical Tips Online, (http://tto.trends.com) (1998)). The 1.0 µl aliquots were used to deduce the yeast transformation efficiency per µg of DNA. From this efficiency the number of colonies on each of the 500 cm² plates was estimated. The yeast colonies were scraped off of each plate into 1-10 ml of YAPD media, spun down by centrifugation, and plasmid DNA prepared using Zymoprep™ (Zymo Research, Orange, CA). From the approximately 50 µl solution of DNA that was prepared, 3 µl was used to electroporate GeneHogs® electrocompetent bacterial cells (Research Genetics, Huntsville, AL). The electroporated bacteria were plated onto LB plates containing kanamycin (5 µg/ml). The number of kanamycin resistant clones was recored. Table 1 shows the results of this experiment.

**Table 1**

| **Condition** | **Yeast cfu/plate** | **KAN cfu/plate** | **Transformation Efficiency** |
|---|---|---|---|
| | | | |
| 1:1 | 163 | lawn | 3.26X10⁶ |
| | 1630 | 77 | |
| | 16,300 | 66 | |
| | 163,000 | 14 | |
| | | | |
| 1:10 | 438 | 119 | 8.76X10⁶ |
| | 4380 | 50 | |
| | 43,800 | 0-arc | |
| | 438,000 | 14-arc | |
| | | | |
| 1:100 | 477 | 68 | 9.54X10⁶ |
| | 4770 | 11 | |
| | 47,700 | 13 | |
| | 477,000 | 39 | |
| | | | |
| 1:1000 | 732 | 11 | 1.46X10⁶ |
| | 7320 | 0 | |
| | 73,200 | 39 | |
| | 732,000 | 296 | |

Since the Zymoprep™ method and bacterial electroporation are both known to be saturable it is not relevant to make predictions of homologous recombination frequency from the absolute *E. coli* kanamycin colony numbers. Instead we use a presence/absence call of kanamycin-resistant colonies from various yeast colony numbers to estimate homologous recombination frequency in yeast. Specifically, since kanamycin resistance was present 4 times out of 4 when the target plasmid was present amongst 100 fold excess of control plasmid, and 3 times out of 4 when the target plasmid was present amongst 1000-fold excess of control plasmid, we estimate that homologous recombination events were present at a frequency between 1 in 75 and 1 in 150, i.e., approximately 1 homologous recombination event in 112 yeast colonies. Fidelity of the recombination event has been verified by restriction analysis of plasmid DNA prepared from the kanamycin resistant colonies. Of 24 kan^{r} colonies isolated all demonstrated correct homologous recombination. We conclude that homologous recombination events reliably serve to identify rare plasmids in a mixture of other plasmids.

In the example shown above, the YTC was targeted into the TK cassette of the pCosRKO backbone. When preparing a targeting cassette for a GRI from a genomic library a YTC is directed to the GRI by making the appropriate chimeric oligonucleotides when PCR-amplifying the YTC. Since the library is divided into 960 pools of 1000 clones/pool, typically only one clone/pool or 1/1000th of the DNA in a positive pool would be a target for the YTC. A Qiagen robot will yield approximately 3 µg of DNA for each pool. One tenth of this DNA (i.e., 300 ng) is used for the yeast electroporation along with 160 ng of the appropriate YTC for the GRI. This will yield at least 450,000 yeast transformants according to the yeast transformation efficiencies shown above. Of these, an estimated 450 will be transformed for the particular clone bearing the GRI. Based on the demonstrated homologous recombination frequency, approximately 4 will have undergone homologous recombination with the YTC and will be recovered as kanamycin resistant *E. coli* colonies. Plasmid DNA from any one of these colonies is suitable to be prepared and used for targeting ES cells.

### EXAMPLE 4 - Characterizing Clones Isolated from a pCosRKO Genomic library

Individual clones identified as kanamycin resistant colonies on the above procedure are isolated and grown overnight in liquid culture at 30 °C in the presence of ampicillin (100 µg/ml) and plasmid DNA prepared by conventional miniplasmid preparation methods known to those skilled in the art. This DNA is then restriction-mapped using the FLASH™ Nonradioactive Gene Mapping Kit (Stratagene Inc., La Jolla, Calif.) according to the procedures described by the manufacturer. Briefly this involves first completely digesting 10µg of the phage DNA with the restriction enzyme Notl using standard restriction enzyme digest conditions (Maniatis *et al., supra*). NotI cuts all clones in the vector DNA at either end of the cloned insert, leaving a T3 bacteriophage promoter attached to one end of the insert and a T7 bacteriophage promoter attached to the other end. The Notl-digested DNA is then partially digested with the enzyme *EcoR*I, as an example, using limiting amounts of enzyme (0.2 units/µg DNA), in an 84 µl reaction volume at 37°C. Aliquots (26 µl) are removed after 3 minutes, 12 minutes, and 40 minutes, and the reaction is stopped by the addition of 1 µl of 0.5M EDTA. DNA from all three time points is resolved on a 0.7% agarose gel, visualized by ethidium bromide staining, and then transferred to a GeneScreen Plus® membrane (NEN Research Products, Boston, Mass.) by capillary transfer (Maniatis *et al.,supra*). The membrane is hybridized with an alkaline phosphatase labeled oligonucleotide that is specific for the T3 promoter (supplied with the FLASH™ kit) using reagents and methods supplied by the kit manufacturer. After hybridization, the membrane is washed and developed with a chemiluminescent-yielding substrate and then exposed to X-ray film in the dark for approximately 60 minutes.

The oligonucleotide probes effectively label one end of the insert. By determining the positions of the bands on the X-ray film and calculating the DNA size to which they correspond, it is possible to determine the position of the *EcoR*I sites relative to the T3 end of the insert. These results are then complemented by stripping the probe off of the membrane, and rehybridizing with a T7-specific oligonucleotide in order to determine the positions of the *EcoR*I sites relative to the T7 end of the insert. This process is repeated using other 6 base pair-recognition site restriction endonucleases.

### EXAMPLE 5 - Using a gene targeting vector generated from a pCosRKO genomic library

### A) Embryonic Stem Cells

The DBA-252 ES cells are created as described (Roach, et al., Exp. Cell Res.,**221**:520-525,1995). The cells are also grown as described therein.

### B) DNA preparation

DNA is prepared for a gene targeting experiment by growing up a preparation of a gene targeting vector clone obtained by the above procedures and isolating DNA using a Qiagen® Maxi Kit (Qiagen, Valencia, CA). 400µg of targeting vector DNA are prepared for electroporation by linearizing the plasmid with either the enzyme Swal or PI-Pspl in a 1 ml reaction volume. The DNA is then precipitated by the addition of ethanol, washed with 70% ethanol, and resuspended in 500 µl of sterile water.

### C) Electroporation of prepared targeting vector DNA into ES cells

The linearized targeting vector DNA is then electroporated into ES cells using a Bio-Rad Gene Pulser™ System (Bio-Rad Laboratories, Hercules, Calif.) as follows. In each of ten electroporation cuvettes, 40 µg of DNA is electroporated into 5 x 10⁶ cells suspended in 0.8 ml ES cell medium. The electroporation conditions are 250 V and 500µF, which typically result in time constants ranging between 5.7-6.2 seconds. After electroporation the cells are incubated for 20 minutes at room temperature in the electroporation cuvettes. All the electroporated cells are then pooled and distributed approximately equally onto 20 tissue culture plates (100 mm x 15 mm).

After 24 hours, the plates are aspirated and fresh ES cell medium is added. The following day, the medium in 19 plates is replaced with ES cell medium supplemented with 150 µg/mL of G418 (Life Technologies, Bethesda, MD) and 0.2 µM gancyclovir (Syntex, Palo Alto, CA). The medium in one plate is supplemented with 150µg/mL of G418 alone. After an additional 6 days, resultant individual ES cell colonies are picked off of the plates and separately expanded in individual wells of 24 well plates as described (Wurst and Joyner, Gene targeting,**126**:33-61, 1993). A comparison of the number of colonies that grow on the plates supplemented with G418 and gancyclovir versus the number that grow on the plates supplemented with G418 alone is used to determine the efficiency of negative selection, which typically between 3-10 fold.

### D) Analyses of the gene-targeted ES cells

When the cell culture in each well of the 24-well plates becomes approximately 80% confluent, the cells are washed with PBS and then dispersed with two drops of trypsin-EDTA. Trypsinization is stopped by the addition of 1 ml of ES cell medium. An aliquot (0.5 mL) of this suspension is transferred to each of two wells of separate 24-well plates. After the cells have grown to near confluence, one of the plates is used for cryopreservation of the cell line while the other is used as a source of DNA for each of the cloned cell lines.

For cryopreservation, the cells in a 24-well plate are first chilled by placing the plate on ice. The medium is then replaced with fresh ES cell medium supplemented with 10% DMSO and 25% FBS. The plate is then cooled at approximately 0.5°C/minute by insulating the plate in a Styrofoam box and placing it in a -70 °C freezer.

To isolate the DNA from the cloned cell lines on the other 24-well plate, the medium in each well is replaced with 500 µl of digestion buffer (100 mM Tris-HCl, pH8.5, 5 mM EDTA, 0.2% SDS, 200 mM NaCI, and 100 µg/ml proteinase K) and incubated overnight at 37°C. After overnight incubation, 500µl of isopropanol is added to each well and the plate is agitated for 15 minutes on an orbital shaker. The supernatant fluid is aspirated and replaced with 500 µl of 70% ethanol and the plate is shaken for an additional 15 minutes. The DNA precipitate is picked out of the well and dissolved in 50 µl of TE solution (10 mM Tris-HCl pH 7.5, 1 mM EDTA).

The analysis for a gene targeting event of the GRI involves a Southern hybridization screen of endonuclease digested ES cell DNA. The enzymes chosen for this procedure are ones that do not cut in one or the other homology arm of the gene targeting vector but do cut in the corresponding flanking MCS of the targeting arm. For example, an enzyme is chosen that does not cut in the left arm of the targeting vector but does cut in the MCS immediately flanking the left arm. Similarly, a second enzyme for a complementary digest would be chosen that does not cut in the right arm but does cut in the MCS immediately flanking the right arm. The probe for this analysis is derived from the bacterial/mammalian positive selection cassette. For the left arm analysis a probe is prepared by isolating the 350 base pair fragment obtained by digesting pDual-NotI with PvuII and Not I. The right arm probe is prepared by isolating the 660 base pair fragment obtained by digesting pDual-Notl with Notl and Cspl.

An aliquot (10 µl) of each DNA sample is digested with the enzyme chosen for analysis of either the right or left arms, resolved on a 0.8% agarose gel, and transferred to a GeneScreen Plus™ membrane. The probe is prepared by first isolating the appropriate band from the double digested pDual-Notl (described above). The probe is labeled with ³²P-dCTP by random priming and hybridized overnight to the membrane at 58°C (Church *et al.,* Proc. Natl. Acad. Sci. USA, **81:**1991-95, 1984). An ES cell line in which the GRI has been successfully targeted yields a fragment that is larger than that predicted by the restriction map of the gene targeting vector. This is the result of homologous recombination removing the MCS from the arm of homology as the targeting vector is incorporated into the genome by homologous recombination, whereas the MCS is not removed when the targeting vector randomly inserts into the genome.

All cell lines scored as putative homologous recombinants by this primary screen are then further screened using an aliquot of DNA digested with the appropriate enzyme to analyze the complementary arm of homology and probed with the alternative probe derived from pDual-Notl. Again, a bona fide homologous recombination event results in a band on the Southern blot that is larger than would be predicted from the restriction map of the gene targeting vector, due to the removal of the MCS for that arm. Cells from the resulting cultures are used to make chimeric mice.

### EXAMPLE 6 - Establishment of Gene-targeted mice homozygous for mutations in the GRI

Gene-targeted ES cells described above are used to make chimeric mice by aggregating the ES cells to E2.5 embryos and transferring the aggregated embryos to pseudopregnant females (See, e.g., Wood et al., Nature, **365**:87-89, 1993). ES cells are prepared for aggregation by limited trypsinization to produce clumps that averaged 10-15 cells. E2.5 embryos are collected from superovulated CD-1 female mice (albino) by oviduct flushing as described by Hogan et al. (Manipulating the Mouse Embryo: A Laboratory Manual, Cold Spring Harbor Laboratory, Cold Spring Harbor, N.Y., 1986; incorporated herein by reference). The zona pellucida is removed from the embryos using acidic Tyrode's solution (Sigma Chemical Co., St. Louis, MO). Aggregation wells are created by pressing a blunt metal instrument (i.e., a darning needle) into tissue culture plastic. Embryos are then placed in a well together with a clump of approximately 10-15 ES cells in a small drop (approximately 20 µl) of M16 medium (Sigma Chemical Co., St. Louis, MO) under mineral oil. After an overnight incubation (37 °C, 100% humidity, 5% CO₂ in air), approximately 20 of the aggregated embryos are transferred to the uterine horns of each pseudopregnant female (Hogan *et al., supra*). Contribution of the ES cells to the offspring is scored by the appearance of pigmented coat color. Pigmented mice are termed chimeric founders. Germline contribution by the ES cells is scored by the appearance of pigmented offspring from a cross between the chimeric founders and CD-1 females.

The germline chimeras are then used to establish lines of mice bearing a copy of the mutation in the GRI. The presence of the gene-targeted mutation in the pigmented offspring is determined using the Southern blot strategy described above with genomic DNA prepared from a tail sample (Hogan *et al., supra*). Mice that are homozygous for the mutation in the GRI are established by crossing 2 heterozygous mice and are identified using the same Southern blot strategy.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the above-described modes for carrying out the invention which are obvious to those skilled in the field of molecular biology or related fields are intended to be within the scope of the following claims.

## Claims

1. A method of preparing a gene vector, said method comprising:
a) transforming yeast cells with a RKO clone and a yeast targeting cassette (YTC), wherein said RKO clone comprises a genomic clone insert, a yeast replication element, a yeast selectable marker, a bacterial origin of replication, optionally a bacterial selectable marker, and optionally a mammalian negative selection marker, and wherein said YTC comprises a bacterial/mammalian positive selection marker flanked by recombinogenic arms;
b) maintaining said yeast cells under conditions wherein said RKO clone and said YTC undergo homologous recombination via said genomic clone insert and said recombinogenic arms to produce a gene targeting vector;
c) selecting transformed yeast cells by their expression of said yeast selectable marker on said gene targeting vector or on said RKO clone;
d) isolating said gene targeting vector and said RKO clone from said selected yeast cells;
e) transforming bacterial cells with said gene targeting vector and said RKO clone;
f) selecting transformed bacterial cells that grow on selective media that is selective for bacterial cells expressing said bacterial/mammalian positive selection marker, thereby selecting for bacterial cells transformed with said gene targeting vector; and
g) isolating said gene targeting vector from said selected bacterial cells.

2. The method of claim 1 wherein said bacterial cells are Escherichia coli.

3. The method of claim 1 wherein said RKO clone is a cosmid and further comprises at least 1 Cos site.

4. The method of claim 1 wherein said RKO clone further comprises a multiple cloning site, and wherein said genomic clone insert is present within said multiple cloning site.

5. The method of claim 1 wherein said YTC further comprises loxP or FRT sites flanking said mammalian positive selection marker.

6. The method of claim 1 wherein said RKO clone comprises a mammalian negative selection marker.

7. The method of claim 1 wherein said YTC is generated by a PCR reaction using chimeric oligonucleotides bearing sequence identity to both the bacterial/mammalian positive selection marker and the GRI.

8. The method of claim 1 wherein said YTC comprises an internal ribosomal entry site (IRES) element that allows protein translation of said bacterial/mammalian positive selection marker in mammalian cells to occur from mRNA transcripts driven by a promoter in the GRI.

9. The method of claim 1 wherein said bacterial/mammalian positive selection marker lacks a polyadenylation site on the 3' end thereof.

10. A method of preparing gene targeted mammalian cells having a targeted gene mutation, said method comprising:
a) transforming mammalian cells with said gene targeting vector of claim 1;
b) maintaining said mammalian cells under conditions wherein said gene targeting vector and the genome of said mammalian cells undergo homologous recombination to produce a gene targeted mammalian cell; and
c) selecting gene targeted mammalian cells wherein homologous recombination has occurred by selecting gene targeted mammalian cells for their expression of said bacterial/mammalian positive selection marker, thereby obtaining gene targeted mammalian cells containing said targeted gene mutation.

11. The method of claim 10 wherein said mammalian cells are stem cells.

12. The method of claim 10 wherein said mammalian cells are embryonic stem cells.

13. The method of claim 10 wherein said RKO clone comprises a mammalian negative selection marker, and said gene targeted mammalian cells are selected for their expression of said bacterial/mammalian positive selection marker and by their non-expression of said mammalian negative selection marker.

14. A method of making gene targeted mice, said method comprising:
a) combining a gene targeted mouse cell according to claim 11 with an early mouse embryo to produce a gene targeted embryonic construct, and
b) introducing said gene targeted embryonic construct into a female host mouse, wherein said gene targeted embryonic construct is allowed to mature into a chimeric live whole mouse, said whole mouse thereby having a genome that includes said targeted gene mutation.

15. A method of making homozygous gene targeted mice, said method comprising cross-breeding male and female mice obtained by the method of claim 14 to produce offspring mice, and selecting offspring mice from said cross-breeding that are homozygous for said targeted gene mutation.

16. A gene targeting vector comprising a yeast replication element, a yeast selectable marker, a bacterial origin of replication, optionally a bacterial selectable marker, optionally a mammalian negative selection marker, and a genomic clone insert containing a bacterial/mammalian positive selection marker inserted therein.

17. The gene targeting vector of claim 16 wherein said gene targeting vector comprises a mammalian negative selection marker.

18. The gene targeting vector of claim 16 further comprising at least 1 Cos site.

19. The gene targeting vector of claim 16 further comprising a multiple cloning site, and wherein said genomic clone insert is present within said multiple cloning site.

20. The gene targeting vector of claim 16 further comprising loxP or FRT sites flanking said mammalian positive selection marker.
